(19) Europäisches Patentamt
European Patent Office
Office européen des brevets

(11) Publication number: 0 219 505 B1

(12) **EUROPEAN PATENT SPECIFICATION**

(45) Date of publication of patent specification: 01.02.89

(51) Int. Cl.$^4$: C 07 C 93/00, C 07 C 91/10

(21) Application number: 85905641.8

(22) Date of filing: 12.11.85

(86) International application number: PCT/GB 85/00514

(87) International publication number: WO/86063 (06.11.86 Gazettee 86/24)

(54) **STEREOSELECTIVE PROCESS AND CHIRAL INTERMEDIATES FOR ARYLOXYDROPANOLAMINES.**

(30) Priority: 20.04.85 GB 8510146

(43) Date of publication of application: 29.04.87 Bulletin 87/18

(45) Publication of the grant of the patent: 01.02.89 Bulletin 89/05

(84) Designated contracting states: AT BE CH DE FR GB IT LI LU NL SE

(56) References cited:
GB-A- 1 591 723
US-A- 4 210 653

(73) Proprietor: SMITH KLINE & FRENCH LABORATORIES LIMITED
Mundells
Welwyn Garden City Hertfordshire, AL7 1EY (GB)

(72) Inventor: MITCHELL, Michael, Barry
5 Churchfield
Harpenden Hertfordshire AL5 1LJ (GB)
Inventor: KITTERINGHAM, John
22 Becketts
Hertford Hertfordshire SG14 2AN (GB)

(74) Representative: Waters, David Martin, Dr. et al
Smith Kline & French Laboratories Ltd. Patent Department Mundells
Welwyn Garden City Hertfordshire AL7 1EY (GB)

EP 0 219 505 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

The present invention relates to a general method for preparing chiral β-adrenoceptor antagonists and to intermediates therefor. β-Adrenoceptor antagonists are useful in the treatment of a range of diseases for example hypertension, angina, myocardial infarction, arrhythmias, thyrotoxicosis, anxiety, migraine, tremor, glaucoma and congestive heart failure.

Many β-adrenoceptor antagonists have the general formula (I) :

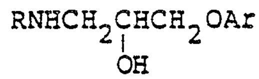
(I)

wherein Ar is an optionally substituted aryl, heteroaryl or heterocyclic group as known in the art, and R is an optionally substituted alkyl group as known in the art for example a group $R^2R^3CH$— as hereinafter defined. The carbon atom bearing the hydroxy group is chiral and it is generally acknowledged that β-adrenoceptor activity resides in the S-isomer. Generally such S-isomers are prepared by resolution or by asymmetric synthesis. However, these processes are difficult and inefficient and, in general, S-isomers of β-adrenoceptor antagonists substantially free of the corresponding R-isomer have not been widely commercialised.

In an asymmetric synthesis known in the art (GB 21305858 A) a compound of the formula (II) :

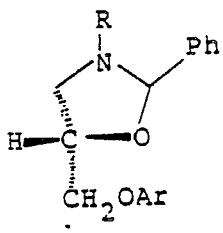
(II)

wherein R and Ar are as hereinbefore defined, is treated with to form the compound of the formula (I). This intermediate compound of the formula (II) is derived from the appropriate isomer of a compound of the formula (III) :

$$RNHCH_2CH\ (OH)\ CH_2OH \qquad (III)$$

wherein R is as hereinbefore defined, by reaction with benzaldehyde and modification of the terminal hydroxy group to introduce the —O—Ar group.

A wide variety of groups R are known in the β-adrenoceptor antagonist art. Clearly the group R is introduced at a very early stage of the overall asymmetric synthesis. Therefore it is particularly inefficient to synthesise chiral compounds as this necessitates a complete synthetic sequence for each value of R.

It is particularly beneficial to have a general intermediate that can provide a wide range of chiral β-adrenoceptor antagonists with introduction of the group R at a late stage of the synthetic sequence. The present invention provides such an intermediate, additionally this is readily formed in good yields.

Accordingly, the present invention provides the S-isomer of a compound of the formula (IV) :

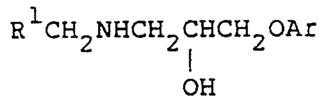
(IV)

wherein Ar is an optionally substituted aryl, heteroaryl or heterocyclic group, and $R^1$ is phenyl optionally substituted by $C_{1-6}$alkoxy or $C_{1-6}$alkyl.

In this specification « S-isomer » relates to the absolute configuration of the pictured carbon atom bearing the hydroxy group ; and is preferably provided substantially free of the corresponding R-isomer.

Ar is an optionally substituted aryl, heteroaryl or heterocyclic group as known in the β-adrenoceptor antagonist art.

Thus, Ar is phenyl optionally substituted by one, two or three groups selected from hydrogen, $C_1$-$C_6$alkyl, $C_{1-6}$alkoxy, $C_{1-6}$alkanoyl, $C_{1-6}$alkanoyloxy, $C_{2-6}$alkenyl, $C_{2-6}$alkenyloxy, $C_{3-10}$cycloalkyl, halo, carbamoyl, $C_{1-6}$alkylcarbamoyl, di-$C_{1-6}$alkylcarbamoyl, arylcarbamoyl, aryl$C_{1-6}$alkyl, aryl$C_{1-6}$alkoxy, $C_{1-6}$-alkanoylamino, aryl$C_{1-6}$alkanoylamino, aryl$C_{1-6}$alkanoyl, aryl$C_{2-6}$alkenyl, carbamoyl$C_{1-6}$alkyl, $C_{1-6}$alkylcarbamoyl-$C_{1-6}$alkyl, di-$C_{1-6}$alkylcarbamoyl$C_{1-6}$alkyl, aryl, aryloxy, $C_{1-6}$alkylthio, cyano, hydroxy-

$C_{1-6}$alkyl, $C_{1-6}$alkanoyl $C_{1-6}$alkyl, amino, $C_{1-6}$alkylamino, di-$C_{1-6}$alkylamino, morpholino, $C_{3-10}$ cycloalkyl $C_{1-6}$alkoxy, $C_{3-10}$cycloalkyl $C_{1-6}$alkoxy $C_{1-6}$alkyl, $C_{3-10}$cyloalkyl $C_{1-6}$alkoxy $C_{1-6}$alkoxy, $C_{1-6}$alkoxy $C_{1-6}$alkyl, $C_{1-6}$alkoxy $C_{1-6}$alkoxy, $C_{3-10}$cycloalkyl $C_{1-6}$alkyl, ureido, $C_{1-6}$alkylureido, $C_{1-6}$ alkylsulfphonamido and arylsulfphonamido. Also Ar is indanyl, indenyl, Enaphthyl, 5-oxotetrahydronaphthyl, 6-oxotetrahydronaphthyl, tetrahydronaphthyl, 5,8-dihydronaphthyl or 7,8-dihydroxytetrahydronaphthyl.

When Ar is an optionally substituted heteroaryl or heterocyclic group it contains up to three heteroatoms selected from oxygen, sulphur and nitrogen. For example, pyridinyl, pyrimidinyl, quinolinyl, indolyl, and 1,2,5-thiadiazol-3-yl. Suitable substituents, where appropriate, are those described hereinabove as suitable for substitution on phenyl.

Examples of groups Ar are 2-cyanophenyl, 2-methylphenyl, 2,3-dimethylphenyl, 4-(2-methoxyethyl)phenyl, 4-(carbamoylmethyl) phenyl, 2-allyloxy, 4-butyronamidophenyl, 2-chlorophenyl, 2-methoxyphenyl, 2-morpholinophenyl, 4-(2-cyclopropylmethoxy)ethyl)phenyl, 4-methanesulphonamidophenyl, 4-(2-acetylethyl) phenyl, 4-morpholino-1,2,5-thiadiazol-3-yl, 4-acetamidophenyl and phenyl.

Suitably $R^1$ is phenyl or phenyl substituted by one, two or three groups selected from $C_{1-6}$alkoxy and $C_{1-6}$alkyl, for example methylphenyl, methoxyphenyl and ethoxyphenyl. Preferably $R^1$ is phenyl.

The compounds of the formula (IV) are useful in that they can be converted to the S-isomers of a wide range of β-adrenoceptor antagonists in two steps.

Accordingly in another aspect the present invention provides a process for preparing the S-isomer of a compound of the formula (V) :

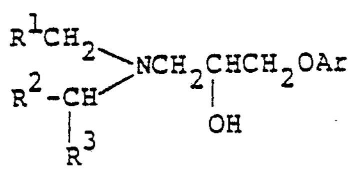

(V)

where $R^1$ and Ar are as hereinbefore defined, and $R^2R^3CH$— represents optionally substituted alkyl : which process comprises reacting the S-isomer of a compound of the formula (IV) as hereinbefore defined with a compound of the formula (VI), or a compound of the formula (VIA) under conditions of reductive alkylation :

$R^2R^3CH—X$ (VI) $R^2R^3C = O$ (VIA)

wherein $R^2$ and R3 are as hereinbefore defined and X is a displaceable group.

In a further aspect the present invention provides the S-isomer of a compound of the formula (V). Values for $R^1$ and Ar are as described for compounds of the formula (IV).

$R^2R^3CH$— is a moiety known in the β-adrenoceptor antagonist art, in which $R^2$ is hydrogen or $C_{1-6}$ alkyl, preferably hydrogen or methyl, and $R^3$ is $C_{1-6}$alkyl optionally substituted by substituents of the following sub-formulae :

i) $R^a—O—C(=O)—$
ii) $R^a—CO—NH—$
iii) $R^a—NR^b—CO—$
iv) $R^a—NR^b—CO—NH—$

wherein $R^a$ is $C_{1-6}$alkyl, phenyl or phenyl $C_{1-6}$alkyl, said phenyl groups being optionally substituted by one, two or three groups as defined hereinabove with reference to substituents for Ar ; and $R^b$ is hydrogen or $C_{1-6}$alkyl. Preferably $R^2R^3CH$— is isopropyl.

The reaction between compounds of the formulae (IV) and (VI) is performed in conventional manner. Suitably X is halo, for example chloro or bromo. Conveniently the reaction is performed in a substantially inert solvent.

The reaction between compounds of the formulae (IV) and (VIA) is performed under conventional reductive alkylation conditions, for example in a substantially inert solvent in the presence of Raney nickel.

In another aspect the present invention provides a process for the preparation of the S-isomer of a compound of the formula (VII) :

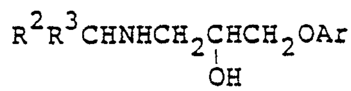

(VII)

wherein $R^2$, $R^3$ and Ar are as hereinbefore defined, which comprises reducing the S-isomer of a compound of the formula (V) as hereinbefore defined.

Suitably the reduction is performed using catalytic hydrogenation in conventional manner. For example using conventional transition metal catalysts such as palladium on a suitable carrier with hydrogen gas or under conditions of catalytic transfert hydrogenation. For example palladium on carbon and palladium hydroxide on carbon may be used. Hydrogenation with hydrogen gas may be performed at non-extreme pressure for example between atmospheric pressure and pressures of 10 atmospheres. Suitably hydrogenation is performed in a $C_{1-4}$alkanol for example ethanol. Catalytic transfer hydrogenation can be effected for example using hydrazine.

The S-isomers of the compounds of the formula(IV) as hereinbefore defined can be prepared by reacting the S-isomer of a compound of the formula (VIII) with acid :

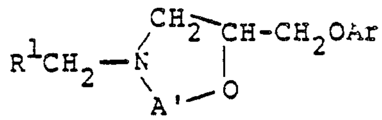

(VIII)

wherein $R^1$ and Ar are as hereinbefore defined, and A′ is an acid-cleavable optionally substituted methylene protecting group for the N— and O— atoms.

Suitably A′ is

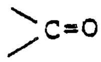

or a group —C($R^4$) ($R^5$) — wherein $R^4$ and $R^5$ are independently $C_{1-6}$alkyl, for example they are both methyl. Suitably also A′ is optionally substituted —CH(Ph)—, preferably A′ is —CH(Ph)—. Suitably such a reaction is performed in an aqueous or mixed aqueous solvent system. Conveniently concentrated hydrochloric acid is used.

The S-isomers of the compounds of the formula (VIII) are conveniently prepared from the S-isomer of a compound of the formula (IX) :

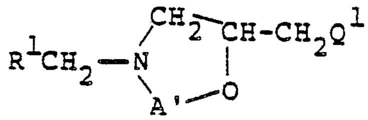

(IX)

wherein A′ and $R^1$ are as hereinbefore defined and $Q^1$ is a displaceable group, and an anion of the formula $^-$OAr wherein Ar is as hereinbefore defined.

In the compounds of the formula (IX) suitably $Q^1$ is a sulphonate such as a $C_{1-6}$alkanesulphonate for example methanesulphonate, trifluoromethanesulphonate or an arylsulphonate for example benzenesulfphonate or toluene-p-sulphonate, or a phosphonate or phosphinate for example diphenylphosphonate or diphenylphosphinate, or $Q^1$ is halo for example bromo or chloro. The anion of the formula $^-$OAr is conveniently formed in situ from the corresponding hydroxy compound. The anion of the formula $^-$OAr is conveniently generated by the action of base, for example an alkali metal hydroxide or alkaline earth metal hydroxide, for example sodium hydroxide, or for example an organic base such as triethylamine. In a suitable alternative the anion of the formula $^-$OAr is introduced into the reaction as the alkali metal salt of the hydroxy compound, for example the sodium or potassium salt. The reaction between the compound of the formula (IX) and an anion of the formula $^-$OAr is conveniently performed in a substantially inert aprotic solvent such as dimethylformamide.

The S-isomers of the compounds of the formula (IX) can be prepared by reacting the S-isomer of a compound of the formula (X) with a compound capable of introducing the group $Q^1$ :

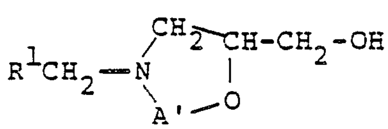

(X)

wherein $R^1$ and A′ are as hereinbefore defined.

Suitable reagents for introducing the group $Q^1$ are sulphonylating agents for example methanesulphonyl chloride, benzenesulphonyl chloride and toluene-p-sulphonyl chloride ; phosphoryl and phosphinyl reagents for example diphenylphosphoryl chloride and diphenylphosphinyl chloride ; and halogenating agents for example thionyl chloride or thionyl bromide, used in conventional manner.

The S-isomers of the compounds of the formula (X) can be prepared in conventional manner by reacting an appropriate precursor, for example a ketone or optionally substituted benzaldehyde, with the S-isomer of a compound of the formula (XI) :

$$R^1CH_2NHCH_2CHCH_2OH \quad (XI)$$
$$\qquad\qquad\quad | \\ \qquad\qquad\quad OH$$

wherein $R^1$ is as hereinbefore defined.

The S-isomers of the compounds of the formula (XI) represent another important intermediate and are a further aspect of the invention. Preferably in such compounds $R^1$ is phenyl.

The S-isomers of the compounds of the formula (XI) can be prepared by reacting the S-isomer of a compound of the formula : $CHOCH(OH)CH_2OH$ with a compound of the formula : $R^1CH_2NH_2$ wherein $R^1$ is as hereinbefore defined, in the presence of a reducing agent, for example hydrogen and a transition metal catalyst.

The following Examples illustrate the invention.

## Example 1

To a stirred suspension of mannitol diacetonide (26.2 g) in benzene (800 ml) was added lead tetraacetate (44.2 g) over 5 minutes. The resultant solid was broken up to give a cloudy solution, stirred for 45 minutes, filtered, evaporated under reduced pressure and distilled to give as an oil, acetone-d-glyceraldehyde (17.7 g), b.p. 44-8 °C/11 mm Hg. To a solution of this in methanol (60 ml), at 5 °C, was added slowly a solution of benzylamine (10.7 g) in methanol (60 ml). This mixture was added to a slurry of 5 % palladium on carbon (1.5 g) in methanol (40 ml) an hydrogenated at 344 KPa (50 p.s.i.) for 30 minutes. The mixture was filtered, treated with 6N hydrochloric acid (150 ml), distilled until the vapour temperature was 98 °C and heated under reflux for one hour. The mixture was cooled, taken to pH 14 with sodium hydroxide, extracted into dichloromethane (3 times), dried and evaporated under reduced pressure to give an oil. This oil was extracted with boiling ether (twice), filtered whilst warm an on standing gave as a crystalline solid (S)-(—)-3-benzylamino-1,2-propanediol (10.1 g), $[\alpha]_D^{25} = -25.72°$ [concentration 1.01 % in ethanol : water : concentrated HCl (17 : 2 : 1)], m.p. 63 °C.

## Example 2

Part of the product from Example 1 (7.24 g) in warm toluene (35 ml) and benzaldehyde (4.66 g) were stirred at room temperature for 90 minutes, then stirred under reflux for 60 minutes removing water using a Dean-Stark apparatus. The solution was then cooled, evaporated under reduced pressure to give an oil which was crystallised from ethyl acetate to give (S)-(—)-2-phenyl-3-benzyl-5-hydroxymethyloxazolidine (8.1 g), m.p. 100 °C.

## Example 3

Part of the product from Example 2 (6.73 g) in pyridine (10 ml) and toluene-p-sulphonyl chloride (4.8 g) were stirred at room temperature for 2 hours (initial cooling). Potassium carbonate (4.13 g) in water (20 ml) was added cautiously and the product extracted into dichloromethane (3 times). The organic extracts were dried and evaporated under reduced pressure to give the tosylate (10.7 g) as an oil. The tosylate in dimethylformamide (12.5 ml) was added in portions to a solution of the anion of 4-(2-(cyclopropylmethoxy)ethyl)-phenol (4.8 g) (formed from sodium hydride (50 % dispersion ; 1.25 g)) in dimethylformamide (12.5 ml). The reaction mixture was heated at 70 °C for 6 hours, poured on to ice (90 g) and extracted into ether (3 times). The ether extracts were washed with water, dried and evaporated to give (S)-(—)-2-phenyl-3-benzyl-5-(4-(2-cyclopropylmethylmethoxy)ethyl)phenoxymethyl) oxazolidine as an orange oil (9.78 g).

## Example 4

The oxazolidine from Example 3 (9.78 g) was slurried in a mixture of water (80 ml) and concentrated hydrochloric acid (20 ml) for one hour until the orange oil had changed to a pale yellow solid. The solid was filtered, washed with water, slurried with ether, filtered and dried under vacuum to give (S)-(—)-2-hydroxy-3-[4-(2-(cyclopropylmethoxy)ethyl)phenoxy]-N-benzylpropylamine as a hydrochloride (5.18 g), m.p. 172 °C (recrystallisation from acetonitrile), $[\alpha]_D^{25} = -14.0°$ [concentration 1.11 % in ethanol : water : concentrated HCl (17 : 2 : 1)], m.p. 172 °C.

## Example 5

S-(—)-2-Hydroxy-3-[4-(2-(cyclopropylmethoxy) ethyl)-phenoxy]-N-benzylpropylamine (1.77 g) and isopropyl iodide (0.98 g) were heated under reflux in ethanol (20 ml) for 12 hours. Ethanol was removed under reduced pressure, the resultant oil dissolved in dichloromethane (20 ml), washed with base (NaOH solution, pH 14, 20 ml), separated, dried ($MgSO_4$), filtered and evaporated to give a pale brown oil. Column chromatography (silica gel : methanol : dichloromethane 1 : 9) gave S-(—)-2-hydroxy-3-[4-(2-(cyclopropylmethoxy)ethyl) phenoxy]-N-benzyl-N'-isopropyl-propylamine as a pale oil (120 mg) ; ($CDCl_3$) 7.30
($5H,m,PhCH_2N$), 3.90 ($2H,m,PhCH_2N$),
($2H,m,CH(OH)—CH_2O—$), 2.98 ($1H,m,NCH(CH_3)_2$), 2.60
($2H,m,NCH_2CH(OH)$), 1.07 ($3H,d,NCH(CH_3)_2$), 1,01
($3H,d,NH(CH_3)_2$), ppm.

Example 6

The product from Example 5 (100 mg) was dissolved in ethanol (10 ml), in the presence of palladium hydroxide on carbon and the mixture hydrogenated at 344 KPa (50 p.s.i.) for 3 hours. Filtration to remove catalyst followed by evaporation of solvent gave a pale oil which was purified by preparative tlc (dichloromethane : methanol (saturated with ammonia) 9 : 1), to give S-(—)-2-hydroxy-3-[4-(2-(cyclopropylmethoxy)ethyl)phenoxy]-N-isopropylpropylamine, 28.5 mg, identical with an authentic sample by chromatography and a nuclear magnetic resonance spectrum.

**Claims**

1. A compound of the formula :

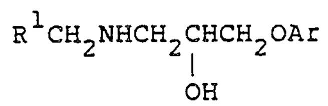

(IV)

wherein $R^1$ is phenyl optionally substituted by $C_{1-6}$alkoxy or $C_{1-6}$alkyl, and Ar is phenyl, indanyl, indenyl, naphthyl, 5-oxotetrahydronaphthyl, 6-oxotetrahydronaphthyl, tetrahydronaphthyl, 5,8-dihydronaphthyl or 7,8-dihydroxytetrahydronaphthyl, or phenyl optionally substituted by one, two or three groups selected from hydrogen, $C_{1-6}$alkyl, $C_{1-6}$alkoxy, $C_{1-6}$alkanoyl, $C_{1-6}$alkanoyloxy, $C_{2-6}$alkenyl, $C_{2-6}$alkenyloxy, $C_{3-10}$cycloalkyl, halo, carbamoyl, $C_{1-6}$alkylcarbamoyl, di-$C_{1-6}$alkylcarbamoyl, arylcarbamoyl, aryl$C_{1-6}$alkyl, aryl$C_{1-6}$alkoxy, $C_{1-6}$alkanoylamino, aryl$C_{1-6}$alkanoylamino, aryl$C_{1-6}$alkanoyl, aryl$C_{2-6}$alkenyl, carbamoyl$C_{1-6}$alkyl, $C_{1-6}$alkylcarbamoyl-$C_{1-6}$alkyl, di-$C_{1-6}$alkylcarbamoyl$C_{1-6}$alkyl, aryl, aryloxy, $C_{1-6}$alkylthio, cyano, hydroxy$C_{1-6}$alkyl, $C_{1-6}$alkanoyl- $C_{1-6}$alkyl, amino, $C_{1-6}$alkylamino, di-$C_{1-6}$alkylamino, morpholino,$C_{3-10}$cycloalkyl$C_{1-6}$alkoxy, $C_{3-10}$cycloalkyl-$C_{1-6}$alkoxy$C_{1-6}$alkyl, $C_{3-10}$cycloalkyl$C_{1-6}$alkoxy$C_{1-6}$alkoxy, $C_{1-6}$alkoxy$C_{1-6}$alkyl, $C_{1-6}$alkoxy$C_{1-6}$alkoxy, $C_{3-10}$cycloalkyl-$C_{1-6}$alkyl, ureido, $C_{1-6}$ alkylureido, $C_{1-6}$alkylsulphonamido and arylsulphonamido ; or Ar is a heteroaryl or heterocyclic group optionally substituted as hereinabove defined, containing up to three heteroatoms selected from oxygen, sulphur and nitrogen : in the form of the S-isomer.

2. A compound of the formula

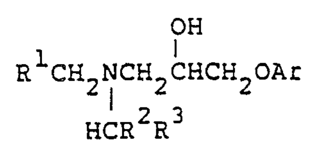

(V)

where $R^1$ is phenyl optionally substituted by $C_{1-6}$alkoxy or $C_{1-6}$alkyl, $R^2R^3CH$— where $R^2$ is hydrogen or $C_{1-6}$alkyl and $R^3$ is $C_{1-6}$alkyl optionally substituted by :

i) $R^a$—O—C(=O)—
ii) $R^a$—CO—NH—
iii) $R^a$—$NR^b$—CO—
iv) $R^a$—$NR^b$—CO—NH—

wherein $R^a$ is $C_{1-6}$alkyl, phenyl or phenyl$C_{1-6}$alkyl, said phenyl groups being optionally substituted by one, two or three groups as defined in claim 1 with reference to the substituents for Ar ; and $R^b$ is hydrogen or $C_{1-6}$alkyl.
and Ar is as defined in claim 1.

3. A compound according to claim 1 or claim 2 wherein $R^1$ is phenyl.

4. A compound according to claim 2 or 3 wherein $R^2R^3CH$— is isopropyl.

5. A compound of the formula (XI) :

$$R^1CH_2NHCH_2\underset{\displaystyle OH}{\underset{|}{C}}HCH_2OH \qquad (XI)$$

wherein $R^1$ is as defined in claim 1 : in the form of the S-isomer.

6. A compound according to claim 5 wherein $R^1$ is phenyl.

**Patentansprüche**

1. Verbindung der Formel

$$R^1CH_2NHCH_2\underset{\displaystyle OH}{\underset{|}{C}}HCH_2OAr \qquad (IV)$$

in der $R^1$ eine gegebenenfalls durch einen $C_{1-6}$-Alkoxy- oder $C_{1-6}$-Alkylrest substituierte Phenylgruppe ist und Ar eine Phenyl-, Indanyl-, Indenyl-, Naphthyl-, 5-Oxotetrahydronaphthyl-, 6-Oxotetrahydronaphthyl-, Tetrahydronaphthyl-, 5,8-Dihydronaphthyl- oder 7,8-Dihydroxytetrahydronaphthylgruppe oder eine Phenylgruppe bedeutet, die gegebenenfalls durch ein, zwei oder drei Reste aus der folgenden Gruppe substituiert ist : Wasserstoffatome, $C_{1-6}$-Alkyl-, $C_{1-6}$-Alkoxy-, $C_{1-6}$-Alkanoyl-, $C_{1-6}$-Alkanoyloxy-, $C_{2-6}$-Alkenyl-, $C_{2-6}$-Alkenyloxy-, $C_{3-10}$-Cycloalkylreste, Halogenatome, Carbamoylgruppen, $C_{1-6}$-Alkylcarbamoyl-, Di-$C_{1-6}$-alkylcarbamoyl-, Arylcarbamoyl-, Aryl-$C_{1-6}$-alkyl-, Aryl-$C_{1-6}$-alkoxy-, $C_{1-6}$-Alkanoylamino-, Aryl-$C_{1-6}$-alkanoylamino-, Aryl-$C_{1-6}$-alkanoyl-, Aryl-$C_{2-6}$-alkenyl-, Carbamoyl-$C_{1-6}$-alkyl-, $C_{1-6}$-Alkylcarbamoyl-$C_{1-6}$-alkyl-, Di-$C_{1-6}$-alkylcarbamoyl-$C_{1-6}$-alkyl-, Aryl-, Aryloxy-, $C_{1-6}$-Alkylthioreste, Cyanogruppen, Hydroxy-$C_{1-6}$-alkyl-, $C_{1-6}$-Alkanoyl-$C_{1-6}$-alkylreste, Aminogruppen, $C_{1-6}$-Alkylamino-, Di-$C_{1-6}$-alkylaminoreste, Morpholinogruppen, $C_{3-10}$-Cycloalkyl-$C_{1-6}$-alkoxy-, $C_{3-10}$-Cycloalkyl-$C_{1-6}$-alkoxy-$C_{1-6}$-alkyl-, $C_{3-10}$-Cycloalkyl-$C_{1-6}$-alkoxy-$C_{1-6}$-alkoxy-, $C_{1-6}$-Alkoxy-$C_{1-6}$-alkyl-, $C_{1-6}$-Alkoxy-$C_{1-6}$-alkoxy-, $C_{3-10}$-Cycloalkyl-$C_{1-6}$-alkylreste, Ureidogruppen, $C_{1-6}$-Alkylureido-, $C_{1-6}$-Alkylsulfonamido- oder Arylsulfonamidoreste ; oder Ar ein Heteroarylrest oder eine gegebenenfalls wie vorstehend angegeben substituierte heterocyclische Gruppe ist, die bis zu drei Heteroatome aufweist aus der Gruppe Sauerstoff-, Schwefel- oder Stickstoffatome ; wobei die Verbindung als S-Isomer vorliegt.

2. Verbindung der Formel

$$R^1CH_2\underset{\displaystyle HCR^2R^3}{\underset{|}{N}}CH_2\overset{\displaystyle OH}{\overset{|}{C}}HCH_2OAr \qquad (V)$$

in der $R^1$ eine gegebenenfalls durch einen $C_{1-6}$-Alkoxy- oder $C_{1-6}$-Alkylrest substituierte Phenylgruppe ist, $R^2R^3CH$— ein Substituent ist, in dem $R^2$ ein Wasserstoffatom oder ein $C_{1-6}$-Alkylrest und $R^3$ ein $C_{1-6}$-Alkylrest ist, der gegebenenfalls substituiert ist durch :

i) $R^a$—O—C(=O)—
ii) $R^a$—CO—NH—
iii) $R^a$—$NR^b$—CO—
iv) $R^a$—$NR^b$—CO—NH—

wobei $R^a$ ein $C_{1-6}$-Alkylrest, eine Phenylgruppe oder ein Phenyl-$C_{1-6}$-alkylrest ist, wobei die Phenylgruppen gegebenenfalls substituiert sind durch eine, zwei oder drei der in Anspruch 1 für die Substituenten von Ar angegebenen Gruppen ; und $R^b$ ein Wasserstoffatom oder ein $C_{1-6}$-Alkylrest ist ; und in der Ar die in Anspruch 1 angegebene Bedeutung hat.

3. Verbindung nach Anspruch 1 oder 2, in der $R^1$ eine Phenylgruppe ist.

4. Verbindung nach Anspruch 2 oder 3, in der $R^2R^3CH$— eine Isopropylgruppe ist.

5. Verbindung der Formel XI

$$R^1CH_2NHCH_2\underset{\displaystyle OH}{\underset{|}{C}}HCH_2OH \qquad (XI)$$

in der $R^2$ die in Anspruch 1 angegebene Bedeutung hat und die als S-Isomer vorliegt.

6. Verbindung nach Anspruch 5, in der $R^1$ eine Phenylgruppe ist.

## Revendications

1. Un composé de formule :

$$R^1CH_2NHCH_2\underset{OH}{C}HCH_2OAr \qquad (IV)$$

dans lequel $R^1$ est phényle optionnellement substitué par $C_{1-6}$alcoxy ou $C_{1-6}$-alkyle et Ar est phényle, indanyle, indényle, naphthyle, 5-oxotétrahydronaphthyle, 6-oxotétrahydronaphthyle, tétrahydronaphthyle, 5,8-dihydronaphthyle ou 7,8-dihydroxytétrahydronaphthyle, ou phényle optionnellement substitué par un, deux ou trois groupes prélevés sur hydrogène, $C_{1-6}$alkyle, $C_{1-6}$alcoxy, $C_{1-6}$alcanoyle, $C_{1-6}$alcanoyloxy, $C_{2-6}$alcénylme, $C_{2-6}$alcényloxy, $C_{3-10}$ cycloalkyle, halo, carbamoyle, $C_{1-6}$alkylcarbamoyle, di-$C_{1-6}$ alkylcarbamoyle, arylcarbamoyle, aryl$C_{1-6}$alkyle, aryl$C_{1-6}$alcoxy, $C_{1-6}$alcanoylamino, aryl$C_{1-6}$alcanoylamino, aryl $C_{1-6}$alcanoyle, aryl$C_{2-6}$alcényle, carbamoyl$C_{1-6}$alkyle, $C_{1-6}$alkylcarbamoyl-$C_{1-6}$alkyle, di-$C_{1-6}$alkylcarbamoyl$C_{1-6}$alkyle, aryle, aryloxy, $C_{1-6}$alkylthio, cyano, hydroxy$C_{1-6}$alkyle, $C_{1-6}$alcanoyl-$C_{1-6}$ alkyle, amino, $C_{1-6}$alkylamino, di-$C_{1-6}$alkylamino, morpholino, $C_{3-10}$cycloalkyl$C_{1-6}$alcoxy, $C_{3-10}$cycloalkyl-alcoxy $C_{1-6}$alkyle, $C_{3-10}$cycloalkyl$C_{1-6}$-alcoxy$C_{1-6}$alcoxy, $C_{1-6}$alcoxy$C_{1-6}$alkyle, $C_{1-6}$alcoxy-$C_{1-6}$alcoxy, $C_{3-10}$cycloalkyl$C_{1-6}$alkyle, ureido, $C_{1-6}$alkylureido, $C_{1-6}$alkylsulfonamido et arylsulfonamido : ou Ar est un hétéroaryle ou un groupe hétérocyclique substitué optionnellement de la manière ci-avant définie, contenant jusqu'à trois hétéroatomes prélevés sur oxygène, soufre et azote : sous la forme de l'isomère-S.

2. Un composé de formule

$$R^1CH_2\underset{HCR^2R^3}{N}CH_2\overset{OH}{C}HCH_2OAr \qquad (V)$$

où $R^1$ est phényle optionnellement substitué par $C_{1-6}$alcoxy ou $C_{1-6}$alkyle. Où $R^2$ est hydrogène ou $C_{1-6}$ alkyle et $R^3$ est $C_{1-6}$alkyle optionnellement substitué par :

i) $R^a$—O—C(=O)—
ii) $R^a$—CO—NH—
iii) $R^a$—$NR^b$—CO—
iv) $R^a$—$NR^b$—CO—NH—

où $R^a$ et $C_{1-6}$alkyle, phényle ou phényl$C_{1-6}$alkyle, lesdits groupes phényle étant optionnellement substitués par un, deux ou trois groupes tels que définis dans la revendication 1 relativement aux substituants pour Ar ; $R^b$ est hydrogène ou $C_{1-6}$alkyle; et Ar est tel que défini dans la revendication 1.

3. Un composé selon la revendication 1 ou 2 dans lequel $R^1$ est phényle.

4. Un composé selon la revendication 2 ou 3 dans lequel $R^2R^2$CH— est isopropyle.

5. Un composé de formule (XI) :

$$R^1CH_2NHCH_2\underset{OH}{C}HCH_2OH \qquad (XI)$$

dans lequel $R^1$ est tel que défini dans la revendication 1 : sous la forme de l'isomère S.

6. Un composé selon la revendication 5 dans lequel $R^1$ est phényle.